# EUROPEAN PATENT SPECIFICATION

(11) **EP 1 991 165 B1**
(45) Date of publication and mention of the grant of the patent: **08.09.2010**
(21) Application number: 07752187.0
(22) Date of filing: 02.03.2007
(51) Int. Cl.: A61F 2/90

(54) **ENDOLUMINAL PROSTHESES FOR TREATING VULNERABLE PLAQUE**
ENDOLUMINALE PROTHESE ZUR BEHANDLUNG VON EMPFINDLICHER PLAQUE
PROTHÈSES ENDOLUMINALES DESTINÉES AU TRAITEMENT DES PLAQUES VULNÉRABLES

(30) Priority: 03.03.2006 US 778400 P
(43) Date of publication of application: 19.11.2008
(73) Proprietor: Prescient Medical, Inc., Doylestown, PA 18901 (US)
(72) Inventor: KULA, John, Birdsboro, PA 19508 (US)
(74) Representative: MacDougall, Alan John Shaw
(86) International application number: PCT/US2007/005471
(87) International publication number: WO 2007/103236

(56) References cited:
- WO-A-96/38101
- WO-A-2006/068944
- US-A- 6 071 308
- US-A1- 2003 125 799
- US-A1- 2004 044 400
- US-B1- 6 208 887
- US-B1- 6 419 693
- US-B1- 6 899 729
- US-B2- 6 923 829

## Description

### FIELD OF THE INVENTION

The invention relates generally to the fields of expandable endoluminal vascular prostheses and their use in treating atherosclerotic lesions.

### BACKGROUND OF INVENTION

Vulnerable plaques, which are sometimes known as high-risk atherosclerotic plaques, include arterial atherosclerotic lesions characterized by a subluminal thrombotic lipid-rich pool of materials contained by and/or overlaid by a thin fibrous cap. Although vulnerable plaques are non-stenotic or nominally stenotic, it is believed that their rupture, resulting in the release of thrombotic contents, accounts for a significant fraction of adverse cardiac events.

U.S. Publication No. 2002/0004679 discloses drug eluting polymer stents for treating restenosis with topoisomerase inhibitors.

U.S. Publication No. 2002/0125799 discloses intravascular stents for the treatment of vulnerable plaque that consist of opposing end ring portions and a central strut portion having a zig-zag configuration that connects with the end portion at apices of the zig-zag structure. The particular zig-zag structure of the stent tends to cause substantial foreshortening upon radial expansion of the device.

U.S. Publication No. 2005/0137678 discloses a low-profile resorbable polymer stent and compositions therefore.

U.S. Publication No. 2005/0287184 discloses drug-delivery stent formulations for treating restenosis and vulnerable plaque.
[0007a] US 2003/125799 discloses an expandable stent for treating an area of vulnerable plaque. The stent has a plurality of cylindrical rings connected by undulating links. The described stent has a high degree of flexibility in the longitudinal direction, yet has adequate vessel wall coverage and radial strength sufficient to hold open an artery or other body lumen.
[0007b] US 2004/0044400 discloses an intravascular stent assembly for implantation in a body lumen, such as a coronary artery. The stent is designed to treat a lesion with vulnerable plaque by reducing the fibrous cap stresses. The disclosed stent includes distal and proximal end sections and a centre section between the distal and proximal end sections. These sections are directly connected or interconnected by a number of interconnecting links placed so that the stent is flexible in the longitudinal direction while providing high degrees of radial strength and vessel scaffolding.
[0007c] WO 96/38101 discloses an implantable intraluminal prosthesis assembly for use in repairing or replacing damaged or diseased portions of a blood vessel. The prosthesis includes a pair of stents at either end of a graft and a plurality of straight struts extending between and interconnecting the stents.

US 6899729 discloses an intravascular stent that is configured for asymmetrical differential deployment to treat vulnerable plaque. The stent is configured so that reduced expansion forces are imparted during deployment to a fibrous cap, thereby reducing the likelihood of cap rupture. The stent includes cylindrical rings connected by links and a cover attached to a portion of the stent to differentially restrict stent expansion when the stent is expanded from a delivery diameter to an implanted diameter, thereby creating asymmetrical circumferential deployment.

Closest prior art WO 2006/068944 discloses a stent having first and second end sections that are connected by sinuate links. Each of the end sections includes a plurality of stent rings. The sinuate struts of the centre section are formed with a plurality of parallel straight sections that are connected by curved sections with an angle of approximate 90° (Fig. 40). This document is relevant for novelty alone under Art. 54(3) EPC.

### SUMMARY OF THE INVENTION

The present invention provides tubular endoluminal prostheses according to the appended claims.

According to one aspect, the present invention provides a tubular prosthesis for the treatment of vulnerable plaque, comprising: two sinuate annular end sections at opposite ends of the prosthesis each consisting essentially of a single sinuate annular form; and a centre section comprising a plurality of sinuate struts having parallel longitudinal axes with each other and with the longitudinal axis of the tubular shape of the prosthesis, wherein the sinuate struts connect the opposite end sections; and wherein each of the sinuate struts is composed of adjacent straight segments and wherein adjacent straight segments within each of the sinuate struts lie at an angle to each other, which angle is approximately 60-deg or approximately 90-deg.

Additional features, advantages, and embodiments of the invention may be set forth or apparent from consideration of the following detailed description, drawings, and claims. Moreover, it is to be understood that both the foregoing summary of the invention and the following detailed description are exemplary and intended to provide further explanation without limiting the scope of the invention as claimed.

### BRIEF DESCRIPTION OF THE DRAWINGS

FIG. 1 shows an example of a prosthesis in which straight struts having longitudinal axes parallel to the longitudinal axis of the tubular shape of the prosthesis connect two sinuate end sections.

FIG. 2 shows an embodiment of a prosthesis according to the invention having sinuate struts with a 60-deg angle between adjacent straight segments and longitudinal axes parallel to the longitudinal axis of the tubular shape of the prosthesis.

FIG. 3 shows an embodiment of a prosthesis similar to that shown in FIG. 2, except that in FIG. 3 the adjacent straight segments of the sinuate struts are separated by a 90-deg angle.

FIG. 4 shows the example of FIG. 1 in a radially expanded state.

FIG. 5 shows an example of a prosthesis having straight struts that are interrupted by s-shaped curves to promote flexibility. In addition, the longitudinal axes of the struts do not perpendicularly intersect the transverse plane of annular end segments (as shown); instead, they are diagonally angled.

### DETAILED DESCRIPTION

The invention provides tubular endovascular prostheses for the treatment of atherosclerotic lesions, including vulnerable plaques, and methods of treatment using the endoprostheses therefor.

One example provides a tubular endovascular prosthesis for the treatment of atherosclerosis lesions such vulnerable plaques, that includes: at least two sinuate annular sections, each having a common central axis; wherein the prosthesis has two opposite ends with one of the sinuate annular sections disposed at each of the ends; and a plurality of struts having parallel longitudinal axes with each other and connecting adjacent sinuate annular sections to each other. The sinuate annular sections resemble a filament or band that undulates back and forth as a path is formed over the surface of a cylinder. The prosthesis is preferably expandable so that its radius can be increased to contact the wall of blood vessel. The prosthesis may be balloon-expandable and/or self-expanding. In one embodiment, the prosthesis is balloon expandable at a pressure 303975 Pa (3 ATMs) or less. In another embodiment, the prosthesis is self-expanding by virtue of being composed of a shape-memory metal alloy or a shape-memory polymer. The endoluminal prostheses of the present invention do not need to have the hoop strength and radial resiliency that is required by conventional stents that are used in conjunction with angioplasty procedures to prevent restenosis. Accordingly, endoprostheses of the invention may have or lack such hoop strength and resiliency, and may be ofa lighter construction than conventional stents.

In the invention, the longitudinal axes of the struts are parallel to the longitudinal axis of the endoprosthesis. The struts may connect to the sinuate annular sections at the centrally-facing curve peaks of the sinuate annular sections.

A related embodiment of the invention provides a tubular endovascular prosthesis for the treatment of atherosclerosis lesions such vulnerable plaques, that includes: two sinuate annular end sections at opposite ends of the endoprosthesis; and a center section including a plurality of struts having parallel longitudinal axes with each other, wherein the struts connect the opposite end sections. Each of the end section may, for example, be formed by a single sinuate form. In one variation of the embodiment, the struts connect to the end sections at peaks of the sinuate form. The longitudinal axes of the struts are parallel to the longitudinal axis of the tubular shape of the prosthesis.

Various aspects of the invention are described below with reference to the appended figures.

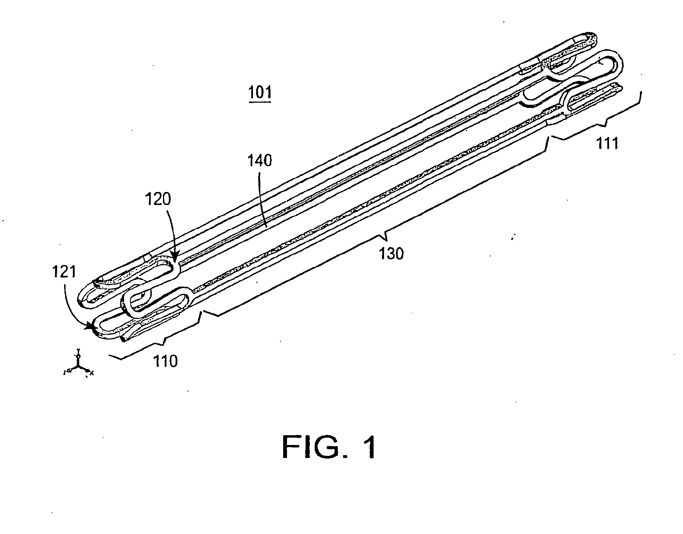

FIG. 1 shows an example of an expandable endoprosthesis 101 that has two sinuate annular end sections 110 and 111 at opposite ends of the device. The end sections 110 and 11 are separated by a central section 130 that is composed of straight struts, for example 140, that connect to the end sections 110 and 111. Each end section has the form of an undulating filament or band that wraps around the shape of a tube as it undulates, thereby forming an annular ring. The outward most part of the curves, for example, 120 and 121, formed by the undulations are referred to as curve peaks herein. The straight struts connect to the more centrally-located curve peaks of the end sections. In the example shown, the longitudinal axes of the struts are parallel to one another and to the central longitudinal axes of the annular end sections. The straight struts of the example attach to the middle of the curve peaks in an essentially perpendicular manner. In the endoprosthesis shown in FIG. 1, every one of the centrally-located curve peaks is connected to an associated strut. However, embodiments in which not every centrally located curve peak has an attached strut are also provided by the invention.

In contrast to the particular zig-zag strut geometry of the stents described in U.S. Publication No. 2003/0125799, the straight strut designs of the above example and various embodiments of the present invention tend to limit foreshortening of the endoprosthesis during radial expansion.

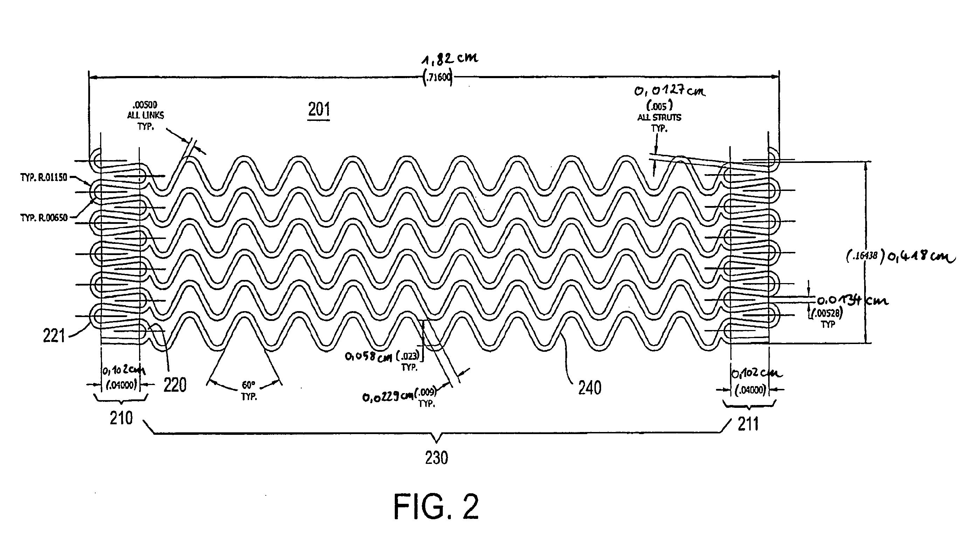

FIG. 2 shows a section of an embodiment 201 of an expandable prosthesis according to the invention in which the struts, for example 240, of the central section 230 are sinuate struts, having a 60-deg angle between adjacent straight segments of the sinuate struts. The dimensions shown in the figure are in inches. The longitudinal axes of the struts are parallel to each other and are also parallel to the longitudinal axis of the tubular shape of the prosthesis. In the embodiment of FIG. 2, the sinuate curves of the different struts of the center section 230 are longitudinally in-phase. The invention also provides embodiments in which at least part of the sinuate curves of struts of the central section are not all in phase. For example, the invention provides an embodiment in which the longitudinal phases of at least two adjacent struts are opposite one another so that a continuous hour glass-shaped path is formed between the adjacent struts. The struts such as 240 connect with the curve peaks of the end sections, but since the struts themselves are sinuate in prosthesis 201, the connection angle is not perpendicular.

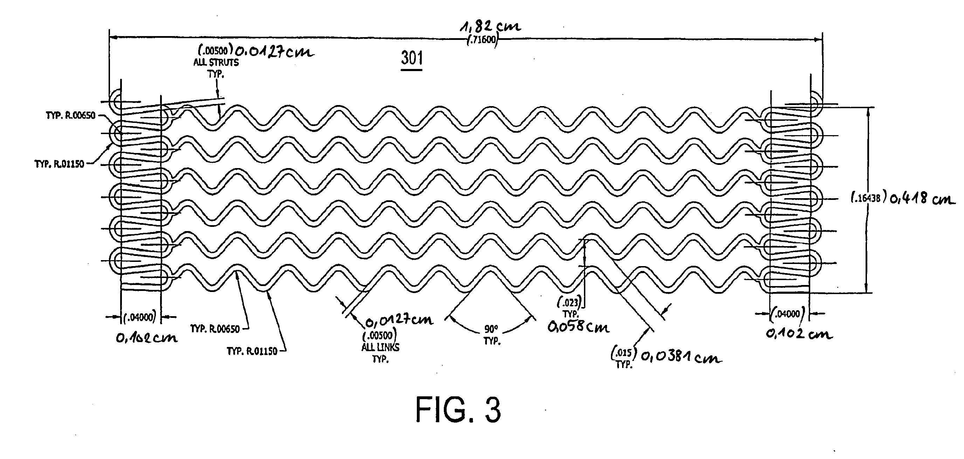

FIG. 3 shows a section of an embodiment of a prosthesis 301 similar to that shown in FIG. 2, except that the sinuate struts of the device in FIG. 3 have a 90-deg angle between adjacent straight segments. The dimensions shown in FIG. 3 are also in inches.

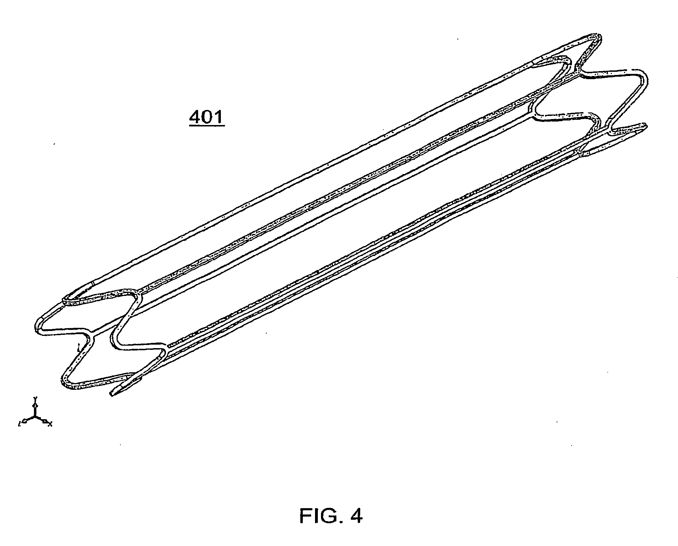

FIG. 4 shows the prosthesis of FIG. 1 in its expanded state 401.

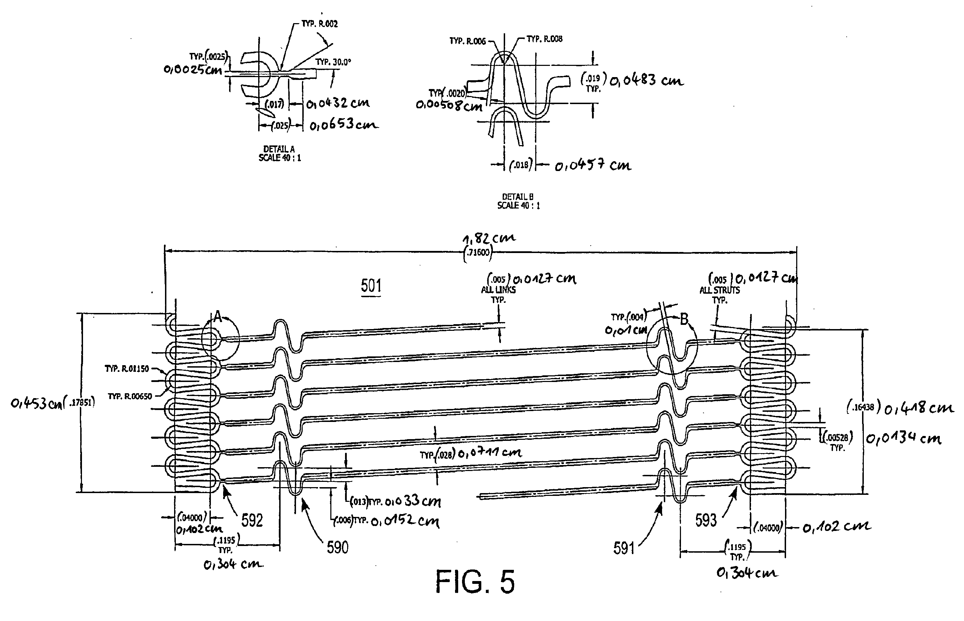

FIG. 5 shows a section of an example prosthesis 501 having straight struts that are interrupted by s-shaped curves, for example 590 and 591, to promote flexibility with respect to the axis of the device. As shown, each strut has two s-shaped segments interrupting the straight form of the strut. Generally, a strut may optionally have one or more sinuate forms along its length in order to promote flexibility of the strut and the overall prosthesis. In addition, the longitudinal axes of the struts in this example do not perpendicularly intersect the transverse plane of annular end segments (as shown); instead they are diagonally angled. Also, the section of a strut directly connecting to a sinuate annular section (a "connecting section"), for example 592 and 593, is narrowed with respect to the main portions of the strut. In one variation the width of the s-shape curve elements and the connecting sections is about 40% of the width of the main portions of the strut. The diagonal struts, sinuate interruptions, and narrowed connecting sections are separate features that may occur together or separately in various embodiments of the invention. The dimensions shown in FIG. 5 are in inches.

The endoprostheses shown in FIGS. 1-5 each have only two sinuate annular sections, which are disposed at the opposing ends of the device. Thus, the invention provides embodiments in which neighboring longitudinal strut elements are not at all, or are at least substantially not, interconnected (to each other) between the sinuate annular end sections. However, the invention also provides embodiments in which there is at least one additional sinuate annular section located between the sinuate annular end sections. In this case, adjacent sinuate annular sections may be connected to each other by struts in the same manner as described above.

The longitudinal length of prostheses according to the invention may, for example, be in the range of 0.5 to 1.0 inch (approximately 1.27 to 2.54 cm), such as 0.716 inch (approximately 1.819 cm). The width of the longitudinal strut elements of any of the embodiment may, for example, be about 0.005 inch (about 0.0127 cm), such as 0.005 inch (approximately 0.0127 cm). When the width of the longitudinal strut elements of the embodiment of FIG. 5 is about 0.005 inch (about 0.0127 em), the width of each of the s-shaped curves, for example 590 and 591, and the narrowed connecting sections, for example 592 and 593, of the strut elements, may for example be about 0.002 inch (about 0.00508 cm).

A further embodiment of the invention provides a method for treating vulnerable plaque in a patient in need thereof that includes the step of deploying any of the prostheses according to the invention at the site of a vulnerable plaque lesion in the patient. Preferably, the strut sections of the device are positioned so that they at least partially traverse a section of blood vessel that has the vulnerable plaque lesion. The deployment involves an expansion of the radius of the device to that the end sections and the strut sections come into contact with the vessel wall. At least one of the strut sections may contact the fibrous cap of the vulnerable plaque and/or at least one strut section may contact the vessel wall in the vicinity of the vulnerable plaque lesion. In either case, contact with the vessel wall promotes endothelialization and remodeling of at least the luminal face of the vulnerable plaque lesion. The invention can be used to promote endothelialization in a region of a blood vessel by deploying a prosthesis according to the invention in the region, irrespective of the underlying pathology of the blood vessel in the region.

The endoprosthesis may be delivered in a decreased radius configuration on a delivery catheter. The endoprosthesis may be crimped on or otherwise position around an inflatable deployment balloon, so that expansion of the balloon at least partially expands the endoprosthesis to its final working radius. For self-expanding versions of the endoprosthesis, use of a delivery balloon is optional. A self-expanding prosthesis may, for example, be restrained in a cylindrical cavity covered by a restraining sheath and deployed by retracting the sheath, as known in the art.

An inflatable deployment balloon of a catheter delivery device may, for example, be at least substantially cylindrically-shaped or it may have a different shape. A deployment balloon that is more expansive or expands with more force in the regions of the annular sinuate segments and less is in the strut segment region(s) may, for example be used, to prevent over-expansion or distension of the struts section. A dumbbell-shaped balloon may, for example, segment in-between, where the enlarged ends of the balloon are designed to expand the annular end segments of the prosthesis. In one method , a single balloon or multiple balloons are used to expand the annular segments but the struts segment(s) is expanded at least predominantly only by expansion of the end segments. In still another method, separate balloons are used to expand the struts segment and the annular segments. In this manner the expansion of struts segments and annular segment can be separately controlled.

Any of the treatment methods of the invention may include a step of locating an atherosclerotic lesion, such as a vulnerable plaque lesion, to be treated by the endoprosthesis in a patient.

Determining the location of a vulnerable plaque or other type of atherosclerotic lesion in a blood vessel of a patient can be performed by any method or combination of methods. For example, catheter-based systems and methods for diagnosing and locating vulnerable plaques can be used, such as those employing optical coherent tomography ("OCT") imaging, temperature sensing for temperature differentials characteristic of vulnerable plaque versus healthy vasculature, labeling/marking vulnerable plaques with a marker substance that preferentially labels such plaques, infrared elastic scattering spectroscopy, and infrared Raman spectroscopy (IR inelastic scattering spectroscopy). U.S. Publication No. 2004/0267110 discloses a suitable OCT system.
Raman spectroscopy-basod methods and systems are disclosed, for example, in: U.S. Patent Nos. 5,293,872; 6,208,887; and 6,690,966; and in U.S. Publication No. 2004/0073120.
Infrared elastic scattering based methods and systems for detecting vulnerable plaques are disclosed, for example, in U.S. Patent No. 6,816,743 and U.S. Publication No. 2004/0111016.
Temperature sensing based methods and systems for detecting vulnerable plaques are disclosed, for example, in: U.S. Patent Nos. 6,450,971; 6,514,214; 6,575,623; 6,673,066; and 6,694,181; and in U.S. Publication No. 2002/0071474.
A method and system for detecting and localizing vulnerable plaques based on the detection of biomarkers is disclosed in U.S. Patent No. 6,860,851. Angiography using a radiopaque and/or fluorescent dye, for example, as known in the art, may be performed before, during and/or after the step of determining the location of the vulnerable plaque, for example, to assist in positioning the prosthesis in a subject artery.

The prostheses of the invention may be metallic and/or polymeric in composition.

Metals used to manufacture a prosthesis according to the invention include, but are not limited to stainless steel, titanium, titanium alloys, platinum and gold. Shape-memory metal alloys may be used to produce self-expanding versions of endoprosthoses according to the invention. For example, suitable shape-memory alloys include, but are not limited, to Nitinol and Elgiloy.

Polymers used for the manufacture of endoprostheses according to the invention may be biodegradable or non-biodegradable. Any suitable sorts of biodegradable polymers and/or biodegradable polymer blends may be used according to the invention. As used herein, the term "biodegradable" should be construed broadly as meaning that the polymer(s) will degrade once placed within a patient's body. Accordingly, biodegradable polymers as referred also include bioerodable and bioresorbable polymers. Suitable types of polymer material include, but are not limited to, polyester, polyanhydride, polyamide, polyurethane, polyurea, polyether, polysaccharide, polyamine, polyphosphate, polyphosphonate, polysulfonate, polysulfonamide, polyphosphazene, hydrogel, polylactide, polyglycolide, protein cell matrix, or copolymer or polymer blend thereof.

Homopolymers of polylactic acid (PLA), for example PLLA, PDLA and poly(D,L,)lactic acid, stereopolymers thereof, and copolymer of PLA with other polymeric units such as glycolide provide a number of characteristics that are useful in a polymeric endoprosthesis for treating a lesion of a blood vessel such as a high risk atherosclerotic plaque (vulnerable plaque). First, polymers made of these components biodegrade in vivo into harmless compounds. PLA is hydrolyzed into lactic acid in vivo. Second, these polymers are well suited to balloon-mediated expansion using a delivery catheter. Third, polymers made of these materials can be imparted with a shape-memory so that polymeric, at least partially self-expanding, tubular endoprostheses can be provided. Self-expanding polymeric prostheses according to the invention may also, for example, be at least partially balloon-expanded. Methods for producing biodegradable, polymeric shape-memory endoprostheses are described, for example, in U.S. Patent Nos. 4,950,258, 5,163,952, and 6,281,262.

Endoprostheses according to the invention may be manufactured by any suitable method. For example, a metallic endoprosthesis can be produced by laser cutting the device from a tubular blank. Methods for forming metallic tubular blacks are well known. For example, sputtering metallic material onto a mandrel may be used. In another example, the shape of the endoprosthesis can be laser cut or stamped out of a flat sheet of metallic material and then formed and welded into a tubular configuration. Once formed into shape, metallic endoprostheses according to the invention may optional be electrochemically polished and/or etched. In one embodiment of the invention, a metallic prosthesis according to the invention is manufactured by separately forming the sinuate annular sections and connecting the struts to the annular sections by, for example, welding or any suitable method for joining metallic components to each other. The sinuate annular sections may be formed separately by, for example, laser cutting from a metallic tubular blank or by winding a filament or band of the metallic material about a suitable cylindrical jig. The ends of such a jig-wound sinuate annular section may be welded together to form a continuous ring structure.

The wall thickness of an endoprosthesis according to the invention may, for example, be in the range of about 20 microns to about 200 microns. In one embodiment, the wall thickness is equal to or less than 200 microns, for example, equal to or less than 125 microns. In one embodiment, the wall thickness is in the range of 20 microns to 125 microns. In another embodiment of the invention, the wall thickness is in the range of 20 to 60 microns. In still another embodiment the wall thickness is in the range of 50 to 100 microns.

A polymeric prosthesis according to the invention, such as one composed of polylactide, may also be laser cut from a tubular blank, such as one formed by extrusion molding.

Endoprostheses according to the invention may be provided with a polymeric, metallic or composite cover that surrounds at least part of the strut sections of the endoprosthesis. In one embodiment, irrespective of the composition of the body of the endoprosthesis, the cover may be polymeric and may, for example, be biodegradable in vivo. The polymer cover may be self-expanding, for example as the result of a shape-memory characteristic. The cover may, for example, be thermoplastically expendable but not be self-expanding. The cover may be porous or non-porous. The cover may, for example, be a continuous porous or non-porous polymeric structure or it may be a braid, woven, or knit polymeric structure. In embodiment in which at least a portion of the strut section is covered, the cover rather than the underlying struts contact the vessel wall upon deployment of the device.

For polymeric endoprostheses, it may also be possible to blend one or more beneficial agents such as drugs with the polymer melt during the formation of an article. Metallic or non-metallic endoprostheses according to the invention may be coated with one or more polymer coatings. The coating(s) may optionally include or be loaded with beneficial agents such as drugs or other compounds useful for treating vulnerable and/or for facilitating the desired functioning of the implanted endoprosthesis, for example, antithrombotic agents such as heparin to inhibit endoprosthesis-induced thrombosis at the treatment site. U.S. Patent No. 5,624,411 teaches methods of coating intravascular stents with drugs.

Although the foregoing description is directed to the preferred embodiments of the invention, it is noted that other variations and modifications will be apparent to those skilled in the art, and may be made without departing from the scope of the invention. Moreover, features described in connection with one embodiment of the invention may be used in conjunction with other embodiments, even if not explicitly stated above.

## Claims

1. A tubular prosthesis (201, 301) for the treatment of vulnerable plaque, comprising:
two sinuate annular end sections (210, 211) at opposite ends of the prosthesis each consisting essentially of a single sinuate annular form; and
a centre section (230) comprising a plurality of sinuate struts (240) having parallel longitudinal axes with each other and with the longitudinal axis of the tubular shape of the prosthesis,
wherein the sinuate struts (240) connect the opposite end sections (210, 211),
wherein each of the sinuate struts (240) is composed of adjacent straight segments and wherein adjacent straight segments within each of the sinuate struts lie at an angle to each other, which angle is approximately 60-deg or approximately 90-deg.

2. The prosthesis of claim 1, wherein the struts (240) connect to the end sections at peaks of the sinuate form.

3. The prosthesis of claim 1, wherein the prosthesis (201, 301) is at least partially metallic.

4. The prosthesis of claim 1, wherein the prosthesis (201, 301) is at least partially polymeric.

5. The prosthesis of claim 1, wherein the prosthesis (201, 301) is at least partially balloon expandable.

6. The prosthesis of claim 1, wherein the prosthesis (201, 301) is balloon expandable to a deployed radius by 303975 Pa or less pressure.

7. The prosthesis of claim 1, wherein the prosthesis (201, 301) is at least partially self-expanding.

8. The prosthesis of any one of the previous claims wherein the angle between the adjacent straight segments is approximately 90-deg.

9. The prosthesis of claim 1, wherein the sinuate struts are in-phase with each other and wherein adjacent struts nest with each other.

## Patentansprüche

1. Eine rohrförmige Prothese (201, 301) zur Behandlung von ungeschütztem Plaque, aufweisend:
a) zwei gebuchtete runde Endbereiche (210, 211) an gegenüberliegenden Enden der Prothese, von denen jeder im Wesentlichen aus einer einzigen gebuchteten runden Form besteht; und
b) einem Mittelbereich (230) umfassend eine Vielzahl von gebuchteten Streben (240) mit parallelen Längsachsen zueinander und zu der Längsachse der rohrförmigen Form der Prothese,
c) wobei die gebuchteten Streben (240) die gegenüberliegenden Endbereiche (210, 211) verbinden,
d) wobei jede der gebuchteten Streben (240) aus benachbarten geraden Segmenten gebildet ist und wobei benachbarte gerade Segmente innerhalb jeder der gebuchteten Streben in einem Winkel zueinander liegen, wobei der Winkel ungefähr 60 Grad oder ungefähr 90 Grad ist.

2. Prothese nach Anspruch 1, wobei die Streben (240) mit den Endbereichen an den Spitzen der gebuchteten Form verbunden sind.

3. Prothese nach Anspruch 1, wobei die Prothese (201, 301) wenigstens teilweise metallisch ist.

4. Prothese nach Anspruch 1, wobei die Prothese (201, 301) wenigsten teilweise polymer ist.

5. Prothese nach Anspruch 1, wobei die Prothese (201, 301) zumindest teilweise mittels eines Ballons erweiterbar ist.

6. Prothese nach Anspruch 1, wobei die Prothese (201, 301) mit einem Ballon mit einem Druck von 303975 Pa oder weniger auf einen Einsatzradius erweiterbar ist.

7. Prothese nach Anspruch 1, wobei die Prothese (201, 301) wenigstens teilweise selbsterweiternd ist.

8. Prothese nach einem der vorgehenden Ansprüche, wobei der Winkel zwischen benachbarten geraden Segmenten ungefähr 90 Grad ist.

9. Prothese nach Anspruch 1, wobei die gebuchteten Streben phasengleich miteinander sind und wobei benachbarte Streben ineinander geschachtelt sind.

## Revendications

1. Prothèse tubulaire (201, 301) pour le traitement de plaques vulnérables, comprenant :
deux sections d'extrémité annulaires sinueuses (210, 211) aux extrémités opposées de la prothèse comprenant chacune essentiellement une seule forme annulaire sinueuse ; et
une section centrale (230) comprenant une pluralité d'entretoises sinueuses (240) ayant des axes longitudinaux parallèles les uns aux autres et avec l'axe longitudinal de la forme tubulaire de la prothèse,
dans lequel les entretoises sinueuses (240) relient les sections d'extrémité opposées (210, 211),
dans lequel chacune des entretoises sinueuses (240) est composée de segments droits adjacents et dans lequel les segments droits adjacents à l'intérieur de chacune des entretoises sinueuses se trouvent à un angle les uns aux autres, lequel angle faisant environ 60 degrés ou environ 90 degrés.

2. Prothèse selon la revendication 1, dans laquelle les entretoises (240) sont reliées aux sections d'extrémité aux sommets de la forme sinueuse.

3. Prothèse selon la revendication 1, dans laquelle la prothèse (201, 301) est au moins partiellement métallique.

4. Prothèse selon la revendication 1, dans laquelle la prothèse (201, 301) est au moins partiellement polymère.

5. Prothèse selon la revendication 1, dans laquelle la prothèse (201, 301) est au moins partiellement expansible par ballon.

6. Prothèse selon la revendication 1, dans laquelle la prothèse (201, 301) est expansible par ballon jusqu'à un rayon déployé par une pression de 303 975 Pa ou moins.

7. Prothèse selon la revendication 1, dans laquelle la prothèse (201, 301) est au moins partiellement auto-expansible.

8. Prothèse selon l'une quelconque des revendications précédentes, dans laquelle l'angle entre les segments droits adjacents fait environ 90 degrés.

9. Prothèse selon la revendication 1, dans laquelle les entretoises sinueuses sont en phase les unes avec les autres et dans laquelle les entretoises adjacentes s'emboîtent les unes avec les autres.
